# EUROPÄISCHE PATENTANMELDUNG

(11) **EP 4 227 291 A1**
(43) Veröffentlichungstag der Anmeldung: **16.08.2023**
(21) Anmeldenummer: 22156144.2
(22) Anmeldetag: 10.02.2022
(51) Int. Cl.: C07C 263/10, C07C 263/20, C07C 265/14

(54) **VERFAHREN ZUR HERSTELLUNG VON ISOCYANATEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die Erfindung betrifft ein Verfahren zur Herstellung von Isocyanaten durch Umsetzung entsprechender Amine mit Phosgen. Außerdem betrifft die Erfindung eine Vorrichtung zur Auftrennung von Abgasströmen aus einer Phosgenierungsreaktion.

## Beschreibung

Die Erfindung betrifft ein Verfahren zur Herstellung von Isocyanaten durch Umsetzung entsprechender Amine mit Phosgen. Außerdem betrifft die Erfindung eine Vorrichtung zur Auftrennung von Abgasströmen aus einer Phosgenierungsreaktion.

Isocyanate sind wichtige Grundstoffe der chemischen Industrie. Sie werden oft in großen Mengen hergestellt. Vor allem Di und Polyisocyanate werden hauptsächlich als Ausgangsstoffe für die Herstellung von Polyurethanen eingesetzt. Ihre großtechnische Herstellung erfolgt in der Regel in guten Ausbeuten durch die Phosgenierung der entsprechenden Amine, Amin-Hydrochloride Amin-Carbonate mit einem Überschuss an Phosgen. Als ein Zwischenprodukt entsteht dabei das entsprechende Carbaminsäurechlorid, welches dann unter Abspaltung von Chlorwasserstoff in das Isocyanat überführt wird.

Als Lösemittel für die Reaktion haben sich in der Praxis Chlorbenzol oder o-Dichlorbenzol etabliert, die sich weitgehend inert verhalten und gut geeignet sind, überschüssiges Phosgen wiederzugewinnen und von Chlorwasserstoff zu trennen. Es ist jedoch auch möglich, andere, unter den Reaktionsbedingungen inerte Lösungsmittel zu verwenden.

Das Prozessabgas, das im Wesentlichen aus Phosgen, Chlorwasserstoff und Lösungsmittel besteht, muss behandelt werden, um eine Freisetzung von Phosgen zu vermeiden. Um die Wirtschaftlichkeit der Verfahren zu verbessern, ist es überdies zweckmäßig, das Prozessabgas, welches im Wesentlichen aus Phosgen, Chlorwasserstoff und Lösungsmittel besteht aufzubereiten, so dass zumindest ein Teil dieser Bestandteile, insbesondere das Phosgen, weiter- oder wiederverwendet werden kann. Für die Trennung dieses Abgasstroms sind zahlreiche Varianten bekannt, die sich je nach Aufgabenschwerpunkt voneinander unterscheiden.

Die US2007/0249859A1 beschreibt eine mindestens 2-stufige Sequenz von Absorptionsschritten, wobei die Sequenz mindestens einen isothermen Absorptionsschritt und mindestens einen adiabatischen Absorptionsschritt umfasst und das auf diese Weise gewonnenen Phosgen in die Phosgenierungsreaktion zurückgeführt wird.

In DE102008009761A1 wird der Prozessabgasstrom zunächst teilweise kondensiert und das flüssige Kondensat in einer Strippkolonne aufbereitet. Dort wird am Sumpf ein flüssiger Lösungsmittelstrom erhalten und am Kopf ein Gasstrom, der zusammen mit den zuvor nicht kondensierten Anteilen in eine Absorption geleitet wird. Dort wird das enthaltene Phosgen in einem Lösungsmittel absorbiert und die erhaltene Phosgen-Lösung wird, gegebenenfalls nach Anreicherung mit weiterem Phosgen, wieder in der Phosgenierungsreaktion eingesetzt.

Auf eine destillative Trennung des Prozessabgases setzt die US2018/0044179A1. Hier wird der Abgasstrom in eine Destillationskolonne geleitet, an deren Sumpf ein phosgenhaltiger Strom entnommen wird. Am Kopf der Kolonne wird ein im Wesentlichen aus Chlorwasserstoff bestehender Strom entnommen, verdichtet und dabei teilweise kondensiert. Der kondensierte Anteil wird entspannt und als Rücklauf am Kopf der Kolonne aufgegeben.

Während Phosgen also häufig in den gleichen Prozess rezykliert wird, bieten sich für Chlorwasserstoff andere Verwertungsmöglichkeiten an. Er kann beispielsweise einfach als wässrige Lösung, also als Salzsäure genutzt oder vermarktet werden. Alternativ kann er katalytisch oder elektrochemisch zu Chlor oxidiert oder in der Oxichlorierung von Ethylen zu Ethylendichlorid genutzt werden.

Viele Isocyanate werden in großen Mengen hergestellt und dabei sind kontinuierliche Verfahren bevorzugt. Isocyanate, die in kleineren Mengen hergestellt werden, werden in der Regel durch chargenweise Flüssigphasenphosgenierung hergestellt, da der Aufwand für eine Umstellung auf ein kontinuierliches Verfahren sehr groß ist und sich angesichts der geringen herzustellenden Mengen nicht bezahlt macht. In einigen Fällen werden auch hybride Prozesse betrieben, in denen eine chargenweise Reaktionsführung mit einer kontinuierlich betriebenen, stromabwärtsgelegenen Aufbereitung der hergestellten Isocyanate kombiniert wird. Dies erfordert die Installation von Pufferbehältern, in die die Reaktionsprodukte abgelassen werden und aus denen dann die kontinuierliche Aufarbeitung gespeist wird.

Auch für die chargenweise Herstellung von Isocyanaten ist eine Behandlung des Abgases und nach Möglichkeit auch die Rückgewinnung von Phosgen aus diesem Abgas wünschenswert. Es ergeben sich allerdings Probleme, auf die im Stand der Technik bisher nicht eingegangen wurde. So sind die Massenströme an Prozessabgas sowie dessen Zusammensetzung je nach Betriebszustand stark schwankend und während es für flüssige Produktströme relativ einfach möglich ist, einen Puffertank zu installieren, und so für einen gleichmäßigen Zulauf-Strom zur Aufarbeitung zu sorgen, ist dies für das Prozessabgas aufgrund der großen Volumina sowie der toxischen und korrosiven Bestandteile nicht einfach möglich.

US4233267A beschreibt ein kombiniertes System aus einem Batch-Reaktor und einer kontinuierlichen Destillationsapparatur, wobei die flüchtigen Reaktionsprodukte zunächst kondensiert und in einem Kondensatbehälter zwischengelagert werden, um von dort aus im Wesentlichen unabhängig von den Betriebsparametern des Batch-Reaktors in die kontinuierlich betriebene Kolonne gespeist zu werden. Für Phosgenier-Prozesse ergeben sich bei dieser Vorgehensweise verschiedene Nachteile. Einerseits erfordert die Kondensation von Phosgen oder gar HCl sehr niedrige Temperaturen. Andererseits erhöht eine solche Vorgehensweise aufgrund der notwendigen Pufferung zwangsläufig die Menge an in der Anlage befindlichem Phosgen.

Für den Betrieb einer Absorption zur Abtrennung von Phosgen aus einem Prozessabgas aus der Isocyanat-Herstellung wurde in der DE102008009761A1 sowie der US2007/0249859A1 jeweils die Empfehlung offenbart, die Menge an Lösungsmittel zur Absorption am Phosgenmassenstrom auszurichten. Dabei Für das Gewichtsverhältnis von Lösungsmittel zu Phosgen am Eintritt in die isotherme Absoption wird der Bereich von 0,1:1 bis 10:1 als bevorzugt und der Bereich von 1:1 bis 3:1 als besonders bevorzugt offenbart.

Überraschend wurde nun jedoch gefunden, dass der Phosgenmassenstrom zur Absorptionsvorrichtung keinen guten Richtwert für den benötigten oder gar optimalen Massenstrom an frischem Lösungsmittel zur Absorptionsvorrichung darstellt. Dies gilt insbesondere bei der chargenweisen Produktion von Isocyanaten durch Phosgenierung der entsprechenden Amine, bei der es zu starken Schwankungen in der Zusammensetzung und im Gesamtmassenstrom des Prozessabgases kommt. Es wurde vielmehr beobachtet, dass auch bei Einhaltung der bevorzugten Bereiche für das Verhältnis aus Lösungsmittel zu Phosgen am Eintritt in die isotherme Absorption es phasenweise zum Durchbruch von Phosgen in das Abgas der Absorptionsvorrichtung kommt, während in anderen Phasen eine unnötig große Menge an Lösungsmittel aufgegeben wurde.

Aufgabe der Erfindung war es also ein verbessertes Verfahren zur Herstellung von Isocyanaten sowie eine Vorrichtung zur Auftrennung von Abgasströmen aus einer Phosgenierungsreaktion bereitzustellen, mit denen sich trotz Schwankungen in Zusammensetzung und Massenstrom des Prozessabgases aus einer Phosgenierung, Phosgen zuverlässig und ressourcenschonend durch Absorption aus dem Prozessabgas abtrennen lässt.

Diese Aufgabe konnte gelöst werden durch ein Verfahren zur Herstellung eines Isocyanats umfassend die Schritte:
A) Umsetzung von mindestens einem Amin oder dessen Salz mit Phosgen, wobei ein flüssiges Reaktionsprodukt enthaltend das Isocyanat sowie ein erster gasförmiger Prozessabgasstrom enthaltend Chlorwasserstoff und Phosgen erhalten werden,
B) Auftrennung des gasförmigen ersten Prozessabgasstroms enthaltend Chlorwasserstoff, Phosgen, gegebenenfalls Lösungsmittel, gegebenenfalls leichtsiedende Nebenkomponenten und gegebenenfalls Inerte in mindestens einer Absorptionsvorrichtung, in der der erste Prozessabgasstrom mit einem Absorptionsmittel kontaktiert wird, wobei eine erste Phosgenlösung mit einem ersten Phosgengehalt und ein an Phosgen abgereicherter zweiter Abgasstrom mit einem zweiten Phosgengehalt erhalten werden,
C) gegebenenfalls Vermischen der in Schritt B) erhaltenen ersten Phosgenlösung mit zusätzlichem Phosgen und/oder Lösungsmittel unter erhalt einer konditionierten zweiten Phosgenlösung,
D) gegebenenfalls Einsatz der ersten Phosgenlösung aus Schritt B) oder der konditionierten zweiten Phosgenlösung aus Schritt C) zur Phosgenierung eines Amins,
E) gegebenenfalls Isolierung des Isocyanats durch destillative Auftrennung des in Schritt A erhaltenen flüssigen Reaktionsprodukts,
dadurch gekennzeichnet, dass
- der erste Phosgengehalt des in Schritt B) erhaltenen, an Phosgen abgereicherten zweiter Abgasstroms mit einem ersten Sollwert für diesen ersten Phosgengehalt verglichen wird und/oder der zweite Phosgengehalt der in Schritt B) erhaltenen Phosgenlösung ermittelt und mit einem zuvor festgelegten zweiten Sollwert für diesen zweiten Phosgengehalt verglichen wird und
- das Absorptionsmittel mit einem Massenstrom M1 in die Absorptionsvorrichtung eingebracht wird und dieser Massenstrom M1 einer Regelung unterliegt, wobei die Führungsgröße für den Massenstrom M1 vorzugsweise automatisch geändert wird, wenn der erste Phosgengehalt und/oder der zweite Phosgengehalt von ihrem jeweiligen Sollwert abweichen.

Bevorzugt bedeuten erfindungsgemäß die Ausdrücke "umfassend" oder "enthaltend", "im Wesentlichen bestehend aus" und besonders bevorzugt "bestehend aus". Die in den Patentansprüchen und in der Beschreibung genannten weiteren Ausführungsformen können beliebig kombiniert werden, sofern sich aus dem Kontext nicht eindeutig das Gegenteil ergibt.

"Mindestens ein", wie hierin verwendet, bezieht sich auf 1 oder mehr, beispielsweise 2, 3, 4, 5, 6, 7, 8, 9 oder mehr. Im Zusammenhang mit Bestandteilen der hierin beschriebenen Verbindungen bezieht sich diese Angabe nicht auf die absolute Menge an Molekülen, sondern auf die Art des Bestandteils. "Mindestens einem Amin" bedeutet daher beispielsweise, dass nur eine Art von Amin oder mehrere verschiedene Arten von Aminen, ohne Angaben über die Menge der einzelnen Verbindungen zu machen, enthalten sein können.

Zahlenwerte, die hierin ohne Dezimalstellen angegeben sind, beziehen sich jeweils auf den vollen angegebenen Wert mit einer Dezimalstelle. So steht beispielsweise "99%" für "99,0%".

Numerische Bereiche, die in dem Format "in/von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

Vorliegend werden unter "leichtsiedenden Nebenkomponenten" Verbindungen verstanden, die bei 10⁵ Pa einen niedrigeren Siedepunkt als das in Schritt A) hergestellte Isocyanat aufweisen. Dies können beispielsweise chlorierte organische Verbindungen oder Fragmente des in Schritt A) hergestellten Isocyanats sein.

In einer bevorzugten Ausführungsform des erfindungsgemäßen Verfahrens liegt der Sollwert für den ersten Phosgengehalt im Bereich von 0,00 Vol.-% bis 2,00 Vol.-%, bevorzugt im Bereich von 0,01 Vol.-% bis 1,00 Vol.-% und besonders bevorzugt im Bereich von 0,02 Vol.-% bis 0,50 Vol.-% und ganz besonders bevorzugt im Bereich von 0,02 Vol.-% bis 0,2 Vol.-%. Ein festgelegter erster Sollwert in diesem Bereich kann mit einem ermittelten Istwert für den ersten Phosgengehalt des in Schritt B) erhaltenen, an Phosgen abgereicherten zweiten Abgasstroms vergleichen werden. Anhand der Abweichung von Sollwert und Istwert kann dann der Massenstrom M1 des Absorptionsmittels in die Absorptionsvorrichtung bevorzugt automatisch angepasst werden.

In einer weiteren bevorzugten Ausführungsform des erfindungsgemäßen Verfahrens liegt der Sollwert für den zweiten Phosgengehalt im Bereich von 20 Gew.-% bis 70 Gew.- %, bevorzugt im Bereich von 30 Gew.-% bis 68 Gew.-% und besonders bevorzugt im Bereich von 42 Gew.-% bis 66 Gew.-%. Ein festgelegter zweiter Sollwert in diesem Bereich kann mit einem ermittelten Istwert für den zweiten Phosgengehalt der in Schritt B) erhaltenen Phosgenlösung vergleichen werden. Anhand der Abweichung von Sollwert und Istwert kann dann der Massenstrom M1 des Absorptionsmittels in die Absorptionsvorrichtung bevorzugt automatisch angepasst werden.

In einer weiteren bevorzugten Ausführungsform des erfindungsgemäßen Verfahrens werden die beiden zuvor beschriebenen Ausführungsformen miteinander kombiniert. Im Normalbetrieb wird in dieser Ausführungsform der zweite Phosgengehalt durch Anpassung des Massenstroms M1 auf den zuvor festgelegten zweiten Sollwert geregelt und bei Überschreitung des nun als Schwellenwert dienenden ersten Sollwerts für den ersten Phosgengehalt, also einer Abweichung vom Normalbetrieb, nämlich dem Durchschlagen einer erhöhten Menge an Phosgen in den zweiten Abgasstrom, wird der Massenstrom M1 erhöht, so dass mehr Absorbens in die Absorptionsvorrichtung eingebracht wird, als für die Einstellung des zweiten Phosgengehalts erforderlich wäre. Die Erhöhung des Massenstroms M1 fällt dabei bevorzugt umso größer aus, je größer die Überschreitung des ersten Sollwerts ist und bleibt entweder für eine bestimmte Zeit aktiv oder bevorzugt bis der ermittelte Istwert für den ersten Phsogengehalt wieder unter den ersten Sollwert gesunken ist. Es handelt sich also um ein sogenanntes Override-Konzept, bei dem während der Zeit des Phosgendurchbruchs die Regelung betreffend des zweiten Sumpfgehalts inaktiv ist und der Massenstrom M1 stattdessen auf Basis des ersten Phosgengehalts vorgegeben wird. Auf diese Weise lässt sich im Normalbetrieb der gewünschte zweite Phosgengehalt der Phosgenlösung möglichst genau einhalten, wobei gleichzeitig ein Durchschlagen von Phosgen in den zweiten Abgasstrom reduziert bzw. verhindert wird.

Beispiele für bevorzugte Diamine sind Diaminotoluol (TDA), insbesondere 2,4-Diaminoluol und 2,6-Diaminotoluol, Diaminodimethylbenzol, Diaminonaphthalin, insbesondere 1,5-Diaminonaphthalin (NDA), Diaminobenzol, insbesondere 1,4-Diaminobenzol (pPDI), Diaminodiphenylmethan (MDA), insbesondere 2,2'-Diaminodiphenylmethan, 2,4'-Diaminodiphenylmethan und 4,4'-Diaminodiphenylmethan, 1,4-Diaminobutan, 1,5-Diaminopentan (PDA), 1,6-Diaminohexan (HDA), 1,11-Diaminoundecan, 1-Amino-3,5,5-trimethyl-5-aminomethylcyclohexan (IPDA), Bis(p-aminocyclohexyl)methan (PACM), 1,5-Diamino-2-methylpentan, 2,5-Diamino-2,5-dimethylhexan, 1,4-Diaminocyclohexan, Hexahydrotoluylendiamin (H6TDA), insbesondere 2,4-Hexahydrotoluylendiamin, 2,6-Hexahydrotoluylendiamin, 1,3-Bis(aminomethyl)benzol (m-XDA), 1,4-Bis(aminomethyl)benzol (p-XDA), Bis(aminomethyl)cyclohexan (H6-XDA), Tetramethylxylylendiamin (TMXDA), Bis(aminomethyl)norbornan (NBDA), Neopentandiamin, 2,4,4-Trimethylhexamethylendiamin, 2,2,4-Trimethylhexamethylendiamin und Mischungen davon.

Beispiele für besonders bevorzugte Amine sind 1,5-Diaminonaphthalin (NDA), 1,4-Diaminobenzol (pPDA), 1,5-Diaminopentan (PDA), 1,6-Diaminohexan (HDA), 1-Amino-3,5,5-trimethyl-5-aminomethylcyclohexan (IPDA), Bis(p-aminocyclohexyl)methan (PACM), 1,5-Diamino-2-methylpentan, 2,5-Diamino-2,5-dimethylhexan, 1,4-Diaminocyclohexan, Hexahydrotoluylendiamin (H6TDA), insbesondere 2,4-Hexahydrotoluylendiamin und 2,6-Hexahydrotoluylendiamin, 1,3-Bis(aminomethyl)benzol (m-XDA), 1,4-Bis(aminometyhl)benzol (p-XDA), Bis(aminomethyl)cyclohexan (H6-XDA) sowie Bis(aminomethyl)norbornan (NBDA) und Mischungen davon.

Beispiele für ganz besonders bevorzugte Amine sind 1,5-Diaminonaphthalin (NDA), 1,4-Diaminobenzol (pPDA), 1,5-Diaminopentan (PDA), Bis(p-aminocyclohexyl)methan (PACM), Hexahydrotoluylendiamin (H6TDA), inbesondere 2,4-Hexahydrotoluylendiamin und 2,6-Hexahydrotoluylendiamin, 1,3-Bis(aminomethyl)benzol (m-XDA) sowie Bis(aminomethyl)norbornan (NBDA) und Mischungen davon.

Beispiele für am meisten bevorzugte Amine sind 1,5-Diaminonaphthalin (NDA), 1,4-Diaminobenzol (pPDA), Bis(p-aminocyclohexyl)methan (PACM), 1,3-Bis(aminomethyl)benzol (m-XDA) sowie Bis(aminomethyl)norbornan und Mischungen davon.

Statt der Amine können auch deren Salze, insbesondere die Hydrochloride oder Carbaminate, vorzugsweise die Hydrochloride der Amine phosgeniert werden. Diese Salze werden dann in der Regel in einem ersten Schritt in situ bei niedriger Temperatur hergestellt, wie beispielsweise in der DE19510259A1 beschrieben, wobei gegebenenfalls auf das Einleiten des Inertgases verzichtet werden kann. Bevorzugt ist es die Amine direkt zu phosgenieren (Basenphosgenierung) und zwar in einer zweistufigen Reaktion bei unterschiedlicher Reaktionstemperatur (Kalt-Heiß-Phosgenierung).

Die Umsetzung kann in Gegenwart eines Lösemittels erfolgen. Im Falle einer Gasphasenphosgenierung wird häufig während der Reaktion selbst auf die Anwesenheit eines Lösungsmittels verzichtet und dieses stattdessen erst nach der Reaktion zugegeben, um die Reaktionsprodukte rasch abzukühlen. Dieser Vorgang wird auch als Quenchen bezeichnet. Es sind alle dem Fachmann bekannten Lösemittel geeignet, die gegenüber den vorherrschenden Reaktionsbedingungen inert sind. Bevorzugte Lösemittel sind ausgewählt aus der Gruppe bestehend aus aromatischen Kohlenwasserstoffen, halogenierten aromatischen Kohlenwasserstoffen, insbesondere chlorierten aromatischen Kohlenwasserstoffen, Estern, Ethern und halogenierten Kohlenwasserstoffen und Mischungen davon. Erfindungsgemäß besonders bevorzugt eingesetzte aromatische Kohlenwasserstoffe sind ausgewählt aus der Gruppe bestehend aus Toluol, Brombenzol, Chlorbenzol, Dichlorbenzol, insbesondere o-Dichlorbenzol, und Mischungen davon. Besonders bevorzugt werden erfindungsgemäß Chlorbenzol, o-Dichlorbenzol oder Mischungen dieser beiden Lösemittel eingesetzt.

Die Umsetzung wird vorzugsweise in kondensierter Phase, also als sogenannte FlüssigphasenPhosgenierung durchgeführt, wobei auch Feststoffe und Gase in der im Wesentlichen flüssigen Reaktionsmischung vorhanden sein können.

Besonders vorteilhaft ist das erfindungsgemäße Verfahren zur Herstellung von Isocyanaten für industrielle Prozesse, in denen die Umsetzung des wenigstens einen Amins oder Amin-Salzes mit Phosgen chargenweise erfolgt. Die chargenweise Herstellung von Isocyanaten, auch als Batch- oder Semi-Batch-Fahrweise bezeichnet, ist gekennzeichnet durch besonders starke Schwankungen sowohl im Gesamtmassenstrom als auch in der Zusammensetzung des Prozessabgases.

In einer weiteren Ausführungsform erfolgt die Umsetzung chargenweise in kondensierter Phase. Unter chargenweiser Umsetzung ist vorliegend zu verstehen, dass das flüssige Umsetzungsprodukt dem Reaktor oder den Reaktoren nicht kontinuierlich entnommen wird, sondern das flüssige Umsetzungsprodukt aus einem Reaktor im Wesentlichen erst dann entnommen und in eine Rohproduktvorlage oder direkt zur weiteren Aufbereitung überführt wird, wenn die Umsetzung zum Isocyanat in diesem Reaktor beendet ist. Dem Fachmann sind verschiedene Methoden bekannt, den Endpunkt der Reaktion zu bestimmen. Meist wird der sogenannte Klarpunkt herangezogen, also der Zeitpunkt, an dem aus der zunächst vorliegenden Suspension bei der Heißphosgenierung eine klare Lösung entstanden ist. Alternativ kann auch das Ende der Entwicklung von HCl-Gas herangezogen werden oder ein konstanter NCO-Gehalt in der Reaktionsmischung. Auch während der Reaktion kann jedoch ein Teil der flüssigen Reaktionsmischung aus dem Reaktor entnommen und wieder in diesen zurückgeführt werden, um beispielsweise eine bessere Durchmischung herbeizuführen oder die Reaktionsmischung zu temperieren, ohne die Bedingung der chargenweisen Phosgenierung im Sinne der vorliegenden Erfindung zu verletzen. Gasförmige Ströme umfassend im Wesentlichen Chlorwasserstoff, Phosgen, Inertgas und Lösungsmitteldämpfe können den Reaktoren hingegen jederzeit, auch kontinuierlich entnommen und aufbereitet werden. Bevorzugt erfolgt die Umsetzung im sogenannten Semi-Batch-Verfahren, auch als Fed-Batch-Verfahren oder Zulauf-Verfahren bezeichnet, das heißt, Amin und/oder Phosgen und gegebenenfalls Inertgas werden ausgehend von einer teilweisen Befüllung des Reaktors während der Reaktion zugefüttert.

Als Reaktoren eigenen sich insbesondere Rührkessel, aber auch andere Reaktorformen, beispielsweise Schlaufenreaktoren, sind prinzipiell einsetzbar. Die Umsetzung erfolgt vorzugsweise bei einem Druck von 1,0 bar(a) bis 20 bar(a), bevorzugt 1,05 bar(a) bis 10 bar(a) und besonders bevorzugt von 1,1 bar(a) bis 6 bar(a). Entsprechend ist der mindestens eine Reaktor vorzugsweise mit einer Druckhalteeinrichtung, beispielsweise einem Regelventil ausgestattet, über die Prozessabgas aus dem Reaktor entweichen kann. Im Falle einer kontinuierlichen Reaktionsführung sind insbesondere Rohrreaktoren besonders geeignet.

In einer weiteren bevorzugten Ausführungsform des erfindungsgemäßen Verfahrens erfolgt die Umsetzung in Form einer Basenphosgenierung. Bei einer Basenphosgenierung, also der Umsetzung der Amine mit Phosgen, wird die Reaktion vorzugsweise zweistufig in dem inerten Lösemittel durchgeführt. Solche Reaktionen sind beispielsweise in W. Siefken, Liebigs Annalen der Chemie, 562 (1949) S. 96 beschrieben. Im ersten Stadium, der Kalt-Phosgenierung, wird die Temperatur der Reaktionsmischung bevorzugt in einem Bereich zwischen 0 und 100 °C gehalten. Es entsteht eine Suspension, die Carbaminsäurechlorid, Aminhydrochlorid und geringe Mengen an freiem Isocyanat enthält. Bevorzugt wird dabei eine Lösung von Phosgen in einem inerten Lösungsmittel vorgelegt und dann eine Lösung oder Suspension des Amins im gleichen Lösungsmittel sowie gegebenenfalls weiteres Phosgen zugegeben. Auf diese Weise wird die Konzentration von freiem Amin gering gehalten und somit die unerwünschte Bildung von Harnstoffen unterdrückt.

Im zweiten Stadium, der Heiß-Phosgenierung, wird die Temperatur erhöht und liegt dann vorzugsweise in einem Bereich von 120 bis 200 °C. Sie wird in diesem Bereich gehalten, während so lange weiteres Phosgen zugeführt wird, bis die Umsetzung zum Isocyanat beendet ist, also die HCl-Entwicklung zum Erliegen kommt und/oder die Reaktionsmischung aufklart. Phosgen wird dabei zweckmäßig im Überschuss verwendet. Bei Bedarf kann die Reaktion sowohl in der Kalt- als auch in der Heißphosgenierung unter Einleitung eines inerten Gases durchgeführt werden.

Sofern es sich bei den umzusetzenden Aminen um hochschmelzende und in dem Lösemittel schlecht lösliche Amine handelt, kann auch eine Suspension des Amins zur Phosgenierung eingesetzt werden. Diese wird vorzugsweise durch Dispergierung mit einem dynamischen Mischaggregat hergestellt, wie in EP2897933B1, Absatz [0020] bis Absatz [0024], beschrieben.

Bei der Amin-Hydrochlorid- oder Carbaminat-Phosgenierung wird das Amin bevorzugt zunächst mit Chlorwasserstoffgas oder Kohlendioxid in einem inerten flüssigen Medium zur Erzeugung des entsprechenden Salzes umgesetzt. Die Reaktionstemperatur liegt während dieser Salzbildung vorzugsweise in einem Bereich von 0 bis 80 °C. Anschließend oder zeitgleich kann bereits eine erste Umsetzung mit Phosgen erfolgen. Dann schließt sich ein weiterer Phosgenierschritt als an, der im Wesentlichen der Heiß-Phosgenierung aus der oben beschriebenen Basenphosgenierung ähnelt und deshalb im Folgenden ebenfalls als Heißphosgenierung bezeichnet wird. Es wird also auch hier die Temperatur bevorzugt im Bereich von 120 bis 200 °C gehalten während Phosgen und optional ein Inertgas in die Reaktionsmischung eingeleitet werden. Die Einleitung erfolgt so lange, bis die Umsetzung zum Isocyanat beendet ist. Auch hier wird Phosgen vorzugsweise im Überschuss eingesetzt, um die Reaktion zu beschleunigen.

Die Phosgenierung erfolgt üblicherweise mit einem Überschuss an Phosgen, das heißt, pro mol Aminogruppen wird mehr als ein mol Phosgen eingesetzt. Bevorzugt beträgt das molare Verhältnis von insgesamt eingesetztem Phosgen zu Aminogruppen 1,02:1 bis 20,0: 1, besonders bevorzugt 1,1:1 bis 10,0:1 und ganz besonders bevorzugt 1,2:1 bis 5,0:1. Gegebenenfalls kann dem Reaktionsgemisch während der Umsetzung weiteres Phosgen oder Phosgenlösung zugeführt werden, um einen ausreichenden Phosgenüberschuss aufrecht zu erhalten bzw. um einen Verlust an Phosgen auszugleichen.

Sowohl bei der Basenphosgenierung als auch bei der Amin-Hydrochlorid- bzw. der Carbaminat-Phosgenierung wird nach Beendigung der Reaktion das verbliebene Phosgen und Chlorwasserstoffgas bevorzugt mit einem Inertgas, vorzugsweise mit Sticktstoff, ausgeblasen. Bei Bedarf kann eine Filtration der Reaktionsmischung erfolgen, um gegebenenfalls vorhandene Feststoffe wie unreagierte Amin-Hydrochloride zu entfernen.

Im Verlauf von chargenweisen Phosgenierungen ergeben sich für jede Charge in der Regel zwei Maxima in Bezug auf den Massenstrom an Prozessabgas. Ein erhöhter Massenstrom an Prozessabgas aus einem Reaktor tritt beispielsweise beim Übergang in die Heißphosgenierung auf. Durch die Erwärmung der Reaktionsmischung sinkt die Löslichkeit von Gasen in der Mischung und es kommt zum Ausgasen gelöster Gase und somit zu einem erhöhten Abgasstrom aus dem Reaktor, der je nach gewähltem Verfahren insbesondere Phosgen, Chlorwasserstoff und/oder Kohlendioxid umfasst. Ein zweites Maximum tritt nach Beendigung der Reaktion auf, wenn verbliebenes Phosgen und Chlorwasserstoff aus der Reaktionsmischung ausgetrieben werden und, sofern die Reaktion unter Druck erfolgte, das Reaktionsgefäß entspannt wird.

Um diese im chargenweisen Prozess auftretenden Schwankungen des Prozessabgasstroms zu verringern, und eine ökonomischere Auslegung der Apparate und Betriebsbedingungen zu ermöglichen, ist es bevorzugt, die Umsetzung in Schritt A) in mindestens 2 parallel angeordneten Reaktoren, besonders bevorzugt in 2 bis 10 parallel angeordneten Reaktoren, ganz besonders bevorzugt in 2 bis 6 parallel angeordneten Reaktoren und am meisten bevorzugt in 2 oder 4 parallel angeordneten Reaktoren durchzuführen, wobei wenigstens ein Reaktor asynchron zu wenigstens einem der anderen Reaktoren betrieben wird. Dies bedeutet, dass das Aufheizen der Reaktionsmischung auf über 100 °C und/oder das Austreiben von verbliebenem Phosgen und Chlorwasserstoff nach Reaktionsende, welches auch gegebenenfalls das Entspannen des Reaktors auf einen niedrigeren Druck umfasst, nicht in allen Reaktoren zeitgleich oder unter gleichen Bedingungen erfolgen.

Es ist beispielsweise beim Übergang in die Heißphosgenierung möglich, die gleiche Temperaturrampe zeitversetzt zu vollziehen oder aber für den nicht synchron mit den anderen Reaktoren betriebenen Reaktor eine andersartige, insbesondere flachere Temperaturrampe zu vollziehen, so dass das damit verbundene Maximum des Abgasmassenstroms geringer ausfällt und/oder nicht mit dem Maximum der Abgasmassenströme der anderen Reaktoren zusammenfällt. Besonders bevorzugt vollzieht der Reaktor die gleiche Temperaturrampe mit einem zeitlichen Versatz, so dass die Temperaturerhöhung im Übergang zu der Heißphosgenierung entweder vor oder nach der Temperaturerhöhung der anderen Rektoren erfolgt. Vorzugsweise beträgt der zeitliche Versatz mindestens 5 Minuten, besonders bevorzugt mindestens 10 Minuten und ganz besonders bevorzugt mindestens 20 Minuten. Ebenfalls vorzugsweise beträgt der zeitliche Versatz maximal 24 h, bevorzugt maximal 8 Stunden, besonders bevorzugt maximal 2 h und ganz besonders bevorzugt maximal 1 h.

Entsprechend ist es auch beim Austreiben von verbliebenem Phosgen und Chlorwasserstoff. Hier können beispielsweise die Entspannung und/oder das optionale Einleiten von Inertgas in mindestens zwei der parallel angeordneten Reaktoren asynchron, also zeitversetzt und/oder mit unterschiedlicher Geschwindigkeit erfolgen, um wiederum zu erreichen, dass das damit verbundene Maximum des Abgasmassenstroms geringer ausfällt und/oder nicht mit dem Maximum der Abgasmassenströme der anderen Reaktoren zusammenfällt. Besonders bevorzugt erfolgt die Entspannung und/oder das optionale Einleiten von Inertgas mit den gleichen Vorgaben bezüglich der zeitlichen Druckprofile bzw. Inertgas-Massenströme wie für die anderen Reaktoren, jedoch mit einem zeitlichen Versatz. Vorzugsweise erfolgt das Austreiben von Phosgen und/oder Chorwasserstoff mit einem zeitlichen Versatz von mindestens 5 Minuten, besonders bevorzugt mindestens 10 Minuten und ganz besonders bevorzugt mindestens 20 Minuten. Ebenfalls vorzugsweise beträgt der zeitliche Versatz maximal 24 h, bevorzugt maximal 8 Stunden, besonders bevorzugt maximal 2 h und ganz besonders bevorzugt maximal 1 h.

In einer weiteren bevorzugten Ausführungsform der Erfindung werden die asynchron betriebenen Reaktoren gemäß der gleichen Fahranweisung betrieben, durchlaufen die einzelnen Prozessschritte jedoch mit einem zeitlichen Versatz von mindestens 5 Minuten, bevorzugt mindestens 10 Minuten und besonders bevorzugt mindestens 20 Minuten. Es ist dabei unerheblich, in welche Richtung der zeitliche Versatz erfolgt, also welcher Reaktor vor- oder nachläuft.

In einer weiteren bevorzugten Ausführungsform wird keiner der parallel angeordneten Reaktoren synchron mit einem der anderen Reaktoren betrieben. Bevorzugt werden alle Reaktoren gemäß der gleichen Fahranweisung betrieben, durchlaufen jedoch die einzelnen Prozessschritte mit einem zeitlichen Versatz zueinander.

Das Prozessabgas enthält in der Regel Phosgen, Chlorwasserstoff, sowie gegebenenfalls Lösungsmitteldämpfe, Inertgas und Spuren von anderen flüchtigen Komponenten der Reaktionsmischung wie beispielsweise Aminen oder Isocyanaten. Dieses Prozessabgas wird in Schritt B) in mindestens einer Absorptionsvorrichtung aufgetrennt in einen an Phosgen abgereicherten Abgasstrom und ein Phosgen enthaltendes Absorbens, also eine Phosgenlösung. Bevorzugt findet die Auftrennung in Schritt B) in genau einer Absorptionsvorrichtung statt.

Geeignete Absorptionsvorrichtungen sind beispielsweise Gaswäscher, bei denen der Prozessabgasstrom mit einem Flüssigkeitsstrom, auch Absorbens oder Waschmedium genannt, in Kontakt gebracht wird, wie beispielsweise Tauchwäscher, Sprühwäscher, Füllkörperkolonnen, Packungskolonnen, Bodenkolonnen oder Fallfilmabsorber. Für eine effiziente Absorption des Phosgens aus dem Prozessabgasstrom können auch verschiedenartige Gaswäscher kombiniert werden. Dabei ist es bevorzugt zwei oder mehr, besonders bevorzugt zwei Gaswäscher in einer Absorptionsvorrichtung zu kombinieren. In einer weiteren bevorzugten Ausführungsform umfasst die Absorptionsvorrichtung einen Gaswäscher ausgewählt aus der Gruppe bestehend aus Füllkörperkolonnen, Packungskolonnen und Bodenkolonnen, bevorzugt bestehend aus Füllkörperkolonnen und Packungskolonnen.

Eine besonders geeignete Absorptionsvorrichtung umfasst einen Fallfilmabsorber, der geeignet ist, eine isotherme Absorption durchzuführen und eine Boden-, Füllkörper- oder Packungskolonne, bevorzugt eine Füllkörper- oder Packungskolonne, besonders bevorzugt eine Packungskolonne, die geeignet ist, eine adiabate Absorption durchzuführen. Bei dem Fallfilmabsorber handelt es sich vorzugsweise um einen vertikal angeordneten Rohrbündelwärmetauscher, wobei das Prozessabgas und das Absorbens sowohl auf der Rohr- als auch auf der Mantelseite des Wärmetauschers, vorzugsweise auf der Rohrseite des Wärmetauschers geführt werden, während auf der jeweils anderen Seite ein Kühlmedium im Gleich- oder im Gegenstrom, vorzugsweise im Gegenstrom zu dem Prozessabgasstrom geführt wird. Das Prozessabgas und das Absorbens können ebenfalls im Gleich- oder im Gegenstrom zueinander, vorzugsweise im Gegenstrom zueinander geführt werden. Die Rohre können optional zur Verbesserung des Stoff- und/oder Wärmeaustauschs mit Füllkörpern, Packungen oder anderen Einbauten versehen werden. Die Boden-, Füllkörper- oder Packungskolonne ist vorzugsweise derart eingerichtet, dass das Prozessabgas aus dem isothermen Gaswäscher im Gegenstrom mit dem Absorbens kontaktiert wird. Das Absorbens wird vorzugsweise am oberen Ende der Kolonne über einen Flüssigkeitsverteiler aufgegeben. Bevorzugt handelt es sich um eine Packungskolonne mit einer strukturierten Packung. Besonders bevorzugt werden der Fallfilmabsorber und die Boden-, Füllkörper oder Packungskolonne in einem kolonnenförmigen Apparat kombiniert, der am unteren Ende eine Zuleitung für das Prozessabgas umfasst. Darüber ist der Fallfilmabsorber angeordnet und über diesem die Boden-, Füllkörper- oder Packungskolonne. Oberhalb des Fallfilmabsorbers sowie am Kopf des kolonnenförmigen Apparats befinden sich vorzugsweise Flüssigkeitsverteiler um das Absorbens gleichmäßig auf die Rohre bzw. die Kolonne zu verteilen. Am Kopf der Absorptionsvorrichtung befindet sich optional ein innen- oder außenliegender Kondensator zur weiteren Kühlung des nun an Phosgen abgereicherten Abgasstroms.

Der möglichst phosgenarme Abgasstrom besteht üblicherweise im Wesentlichen, also zu mindestens 80 Vol.-%, bevorzugt zu mindestens 95 Vol.-% und besonders bevorzugt zu mindestens 98 Vol.-% aus Chlorwasserstoff. Er enthält darüber hinaus Reste des Lösemittels, gegebenenfalls Inertgase und Spuren von Phosgen. Der Phosgengehalt dieses Abgasstroms liegt vorzugsweise bei maximal 1,0 Vol.-%, besonders bevorzugt bei maximal 0,5 Vol.-% und ganz besonders bevorzugt maximal 0,2 Vol-%. Die natürliche und grundsätzlich erstrebenswerte Untergrenze für den Phosgengehalt in diesem Abgasstrom ist 0,00 Vol-%. Das Einhalten dieser Grenze kann aber einerseits zu einem erhöhten Verbrauch an Absorbens führen und andererseits kann es für den Aufbau einer Regelung vorteilhaft sein, einen geringen Phosgengehalt im Abgas zuzulassen, z.B. um diesen als Regelungsgröße zu verwenden. Deshalb liegt der Phosgengehalt im Abgasstrom vorzugsweise bei mindestens 0,01 Vol-%, besonders bevorzugt bei mindestens 0,02 Vol.-% und ganz besonders bevorzugt bei mindestens 0,05 Vol-%.

Um eine möglichst effiziente Absorption zu erreichen, ist es vorteilhaft, das Prozessabgas zunächst auf eine Temperatur im Bereich von 5 °C bis -25 °C bevorzugt im Bereich von 0 °C bis -20 °C, besonders bevorzugt im Bereich von -5 °C bis -15 °C abzukühlen. Die Abkühlung kann einfach mittels eines oder mehrerer Wärmetauscher erfolgen oder durch Quenchen, also indem das Prozessabgas mit einer bereits gekühlten Flüssigkeit in einer Quench-Vorrichtung kontaktiert wird. Dies kann beispielsweise in einem Gaswäscher, vorzugsweise einem Sprühwäscher, oder in einem berieselten Wärmetauscher erfolgen. Als gekühlte Flüssigkeit eignet sich beispielsweise das Absorbens. Vorzugsweise wird das Absorbens dabei nach dem Kontakt mit dem Prozessgas als Flüssigkeit abgeschieden und diese Flüssigkeit in die Quench-Vorrichtung rezirkuliert, wobei der Flüssigkeitskreislauf oder die Quenchvorrichtung selbst einen Kühler enthält, um die Flüssigkeit auf die gewünschte Temperatur abzukühlen. Während der Abkühlung kommt es zur teilweisen Kondensation bzw. Absorption von Phosgen.

Die verbleibende Gasphase wird dann, vorzugsweise im Gegenstrom, mit dem Absorbens in Kontakt gebracht. Dabei wird bevorzugt die aufsteigende Gasphase im Gegenstrom mit dem absteigenden flüssigen Absorbens kontaktiert. Geeignete Vorrichtungen hierfür sind dem Fachmann bekannt. Vorzugsweise handelt es sich bei der Absorption um eine Kombination aus zwei Absorptionsschritten, wobei das Gas bevorzugt zunächst eine isotherme Absorption und dann eine adiabate Absorption durchläuft. Der Mengenstrom des Absorbens ist vorzugsweise variabel und kann jeweils an die vorherrschenden Prozessbedingungen und die daraus resultierenden Erfordernisse angepasst werden. Das Absorbens ist vorzugsweise ausgewählt aus der Gruppe bestehend aus aromatischen Kohlenwasserstoffen, halogenierten aromatischen Kohlenwasserstoffen, insbesondere chlorierten aromatischen Kohlenwasserstoffen, Estern, Ethern und halogenierten Kohlenwasserstoffen oder Mischungen davon. Besonders bevorzugt werden erfindungsgemäß Chlorbenzol, o-Dichlorbenzol oder Mischungen dieser beiden Lösemittel als Absorbens eingesetzt. Ganz besonders bevorzugt handelt es sich bei dem Absorbens um Chlorbenzol.

In einer weiteren bevorzugten Ausführungsform wird in dem mindestens einen Absorptionsschritt ein Lösemittel als Absorptionsmittel eingesetzt, das bevorzugt das Lösemittel ist, welches auch in der Umsetzung des mindestens einen Amins oder dessen Salzes zugegen ist. In der bevorzugten Ausführungsform mit zwei Absorptionsschritten wird frisches oder aufbereitetes Absorbens bevorzugt im zweiten Absorptionsschritt, also dem Absorptionsschritt, den das Gas später durchläuft, eingebracht, während im ersten Absorptionsschritt bevorzugt bereits beladenes Absorbens aus dem zweiten Absorptionsschritt, gegebenenfalls zusammen mit frischem Absorbens und/oder einem zurückgeführten, ebenfalls bereits beladenen Absorbens aus dem ersten Absorptionsschritt und/oder dem vorausgehenden Abkühlvorgang genutzt wird. Bevorzugt durchläuft der Gasstrom nach dem Kontakt mit dem Absorbens einen Kondensator, um aufgenommenes Absorbens zu kondensieren und möglichst vollständig aus dem Gasstrom abzutrennen.

Das beladene Absorbens kann, wie zuvor beschrieben, als Quench-Medium eingesetzt werden, um den Prozessabgasstrom zunächst abzukühlen. Ein Teil des Absorbens wird entweder kontinuierlich oder diskontinuierlich entnommen und, gegebenenfalls nach Anreicherung mit weiterem Phosgen, in einem der Reaktoren zur Phosgenierung des Amins oder dessen Salzes eingesetzt. Bevorzugt handelt es sich bei dem entnommenen, beladenen Absorbens um eine Lösung enthaltend 20 Gew.- % bis 70 Gew.-% , besonders bevorzugt 30 Gew.-% bis 68 Gew.-% und ganz besonders bevorzugt 42 Gew.-% bis 66 Gew.-% Phosgen in Chlorbenzol. Der Gehalt der Lösung hängt dabei insbesondere von dem Phosgengehalt des Prozessabgases, von der Menge an frischem Absorbens zur Absorption und der Menge an entnommenem Absorbens ab.

Die erfindungsgemäß beschriebenen Regelungen in der Vorrichtung können beispielsweise computergestützt durchgeführt werden. Daher ist ein weiterer Gegenstand der Erfindung eine Vorrichtung zur Auftrennung von Abgasströmen aus einer Phosgenierungsreaktion umfassend mindestens eine, bevorzugt genau eine Absorptionsvorrichtung zur Kontaktierung eines ersten Prozessabgasstroms enthaltend Chlorwasserstoff, Phosgen mit einem Absorptionsmittel sowie umfassend einen Computer zur Regelung der Auftrennung, dadurch gekennzeichnet, dass der Computer zur Kontrolle des erfindungsgemäßen Verfahrens eingerichtet ist.

Die nachfolgenden Beispiele dienen zur Verdeutlichung der vorliegenden Erfindung, sollen aber keinesfalls als Einschränkung des Schutzbereichs verstanden werden.

### Beispiele

### Vergleichsbeispiel 1:

In einem 1 m³ Rührkesselreaktor mit Temperiervorrichtung und aufgesetztem Rückflusskühler wurde bei 0° C eine Lösung von 120 kg Phosgen in 300 kg Chlorbenzol hergestellt. Unter Rühren wurde zu der Phosgenlösung eine Suspension von 50 kg 1,5-Diaminonaphthalin in 150 kg Chlorbenzol zugegeben. Ohne weitere Kühlung wurde die Suspension für ca. 120 min gerührt, bevor der Übergang von der Phase der Kaltphosgenierung in die Phase der Heißphosgenierung erfolgte. Dazu wurde die Reaktionsmischung erhitzt und dann etwa 6 h bei Rückfluss gehalten. Der Druck während der Reaktion betrug ca. 2,9 bar(a). Nach dem Aufklaren der Reaktionsmischung, das das Ende der Phosgenier-Rekation anzeigt, wurden die Reaktoren zunächst entspannt. Dann wurde das flüssige Rohprodukt abgelassen und das Isocyanat in einer Destillationssequenz isoliert.

Das Abgas wurde während des Produktionslaufs einer zweistufigen Absoprtionskolonne zugeführt, in der es im Gegenstrom mit Chlorbenzol gewaschen wurde. Das Prozessabgas durchlief dabei zunächst eine isotherme Absorptionsstufe bei ca. -15 °C und anschließend eine adiabate Absorptionsstufe, bevor es als Abgas den Prozess verließ. Frisches Chlorbenzol wurde mit -5 °C am oberen Ende der adiabaten Absorptionsstufe aufgegeben. Der Massenstrom an frischem Chlorbenzol zur Absorptionskolonne betrug ca. 20 kg/h. Nach dem Durchlaufen der adiabaten Absorptionsstufe wurde die dort erhaltene Lösung aus Phosgen in Chlorbenzol als Waschlösung in die isotherme Absorptionsstufe weitergeleitet, so dass letztlich am Sumpf der zweistufigen Absorptionskolonne eine Lösung von Phosgen in MCB entnommen wurde, die nach Anpassen der Phosgenkonzentration als Ausgangsmaterial für weitere Phosgenierungen diente. Die Konzentration der entnommenen Phosgenlösung fiel im Laufe der Reaktion ab, so dass für die Anpassung der Phosgenkonzentration für den erneuten Einsatz eine steigende Menge frisches Phosgen zugesetzt werden musste.

Der Prozessabgasmassenstrom aus dem Reaktor durchlief beim Übergang in die Heißphosgenierung ein Maximum von 70 kg/h und das Prozessabgas bestand zu ca. 90% aus Phosgen und zu ca. 10% aus Chlorwasserstoff. Das Verhältnis von Chlorbenzol zu Phosgen am Eintritt in die Absorptionskolonne betrug zu diesem Zeitpunkt entsprechend ca. 0,3:1 und es wurde am Austritt der Absorptionskolonne ein von Phosgen befreiter Chlorwasserstoffstrom erhalten. Im weiteren Verlauf des Batches fiel der Gesamtmassenstrom an Prozessabgas ab und die Zusammensetzung verschob sich hin zu höheren Chlorwasserstoffgehalten. Überraschend trat in dieser Phase ein Durchschlag von Phosgen ins Abgas der Absorptionskolonne auf. Es wurden 5,2 Vol.-% Phosgen im Abgasstrom per IR-Messung festgestellt. Das Verhältnis von Chlorwasserstoff zu Phosgen am Eintritt in die Absorptionskolonne betrug zu diesem Zeitpunkt ca. 1,3:1.

Am Ende der Reaktion kam der Prozessabgasstrom zum Erliegen. Lediglich beim Entspannen der Reaktoren trat noch einmal für kurze Zeit ein erhöhter Massenstrom an Prozessabgas in der Sammelleitung auf, der jedoch weit weniger ausgeprägt war als das erste Maximum.

Das Vergleichsbeispiel 1 zeigt deutlich, dass mit einem konstanten MCB-Massenstrom in die Absorptionsvorrichtung Probleme im Betrieb dieser Vorrichtung auftreten, nämlich einerseits eine starke Schwankung der Zusammensetzung der erhaltenen Phosgenlösung und andererseits ein zeitweiser Durchschlag von Phosgen ins Abgas der Absorptionsvorrichtung. Dies gilt, obwohl die Mengenverhältnisse von Phosgen zu MCB am Eintritt in die Absoprtionsvorrichtung den im Stand der Technik empfohlenen Mengenverhältnissen entsprach.

### Vergleichsbeispiel 2:

Die Synthese aus Vergleichsbeispiel 1 wurde unter gleichen Reaktionsbedingungen wiederholt. Anders als in Vergleichsbeispiel 1 wurde die Absorptionsvorrichtung hier nicht mit einem konstanten Massenstrom an Chlorbenzol beaufschlagt. Dieser wurde stattdessen in Abhängigkeit vom Phosgenmassenstrom im Prozessabgas variiert, so dass der Massenstrom an frischem Chlorwasserstoff in die Absorptionskolonne stets dem 1,3-fachen des Massenstroms an Phosgen in die Absorptionskolonne entsprach, sofern nicht die Mindest-Berieselungsdichte der Absorptionskolonne unterschritten wurde. So betrug der maximale Massenstrom an Chlorbenzol beim Übergang von der Kalt- in die Heißphosgenierung, also zum Zeitpunkt des größten Phosgenmassenstroms im Prozessabgas, 82 kg/h.

Während der Heißphosgenierung kam es erneut zu einem deutlichen Durchbruch von Phosgen in das Abgas der Absorptionsvorrichtung. Es wurden auch in diesem Versuch über 5 Vol.-% Phosgen im Abgas detektiert. Über die Gesamtlaufzeit der Batch-Produktion war der Verbrauch an Chlorbenzol höher als in Beispiel 1, so dass sich im Mittel eine geringere Konzentration der am Sumpf der Kolonne erhaltenen Phosgenlösung einstellte und entsprechend mehr Phosgen zugesetzt werden musste, um die gewünschte Phosgenkonzentration zu erhalten.

### Beispiel 1 (Erfindungsgemäß):

Die Synthese aus Vergleichsbeispiel 1 wurde wiederholt, wobei zum Betrieb der Absorptionskolonne eine Regelung eingerichtet wurde, die den Massenstrom an Absorbens kontrolliert. Hierzu wurde der Phosgengehalt im Abgas aus der Absorptionsvorrichtung mittels einer Online-Messung verfolgt. Der Zielwert für den Phosgengehalt wurde auf 0,05 Vol-% gesetzt und die Abweichung des gemessenen Werts von diesem Zielwert wirkte über einen Regelkreis letztlich auf ein Regelventil in der Zuleitung des frischen Chlorbenzols, um den Chlorbenzol-Massenstrom zur Absorptionskolonne entsprechend anzupassen und die Abweichung so gering wie möglich zu halten.

Über die Gesamtlaufzeit der Batch-Produktion konnte auf diese Weise unter minimalem Chlorbenzol-Einsatz erreicht werden, dass das Abgas aus der Absorptionskolonne nur minimal mit Phosgen belastet war. Der beobachtete Phosgengehalt schwankte zwischen 0,03 Vol.-% und 0,08 Vol.-%. Eine weitere Nachbehandlung zur Vernichtung des Restphosgens war damit einfach möglich. Die Konzentration der Phosgenlösung am Sumpf schwankte, nahm aber im Durchschnitt den maximal möglichen Wert an, der ohne Durchschlagen größerer Mengen von Phosgen ins Abgas erreichbar war.

### Beispiel 2 (Erfindungsgemäß):

Die Synthese aus Beispiel 1 wurde mit einem anderen Regelungskonzept wiederholt. Bei dieser Regelung lag das Hauptaugenmerk auf einem konstanten Phosgengehalt der am Sumpf der Absorptionskolonne gewonnenen Phosgenlösung (Sumpfkonzentrationsregelung). Dazu wurde diese Konzentration als Regelgröße ermittelt und mit dem angestrebten Sollwert verglichen. Diese Regeldifferenz wurde ausgewertet und automatisch der Massenstrom an MCB zur Absorptionskolonne angepasst, so dass die Differenz minimiert wurde. Zusätzlich wurde ein Override-Konzept implementiert. Dazu wurde für den Phosgengehalt im Abgas nach der Absorptionskolonne in diesem Fall ein Schwellenwert von 0,05 Vol.-% gesetzt, bei dessen Überschreitung die Sumpfkonzentrationsregelung deaktiviert wurde und der MCB-Massenstrom zur Absorptionskolonne um einen vordefinierten Wert erhöht wurde, bis der Phosgengehalt des Abgases wieder unter den Schwellenwert gefallen war.

Diese Betriebsweise führte zu einem sehr konstanten Phosgengehalt der am Sumpf gewonnenen Phosgenlösung von ca. 55 Gew.-%. Während der Heißphosgenierung aktivierte sich für wenige Minuten der Override Modus, so dass der erhöhte Massenstrom an Absorbens zu einem Abfall des Phosgengehalts führte, der durch Zugabe von weiterem Phosgen in die Phosgenlösung kompensiert wurde. Im Gegensatz zu Vergleichsbeispiel 2 konnte mit Hilfe des Overrides der Phosgengehalt im Abgas unterhalb von 0,2 Vol.-% gehalten werden.

### Beispiel 3 (Erfindungsgemäß):

Im Unterschied zu den zuvor beschriebenen Beispielen und Vergleichsbeispielen wurde die Reaktion in Beispiel 3 in zwei gleich ausgestatteten, parallel angeordneten Reaktoren durchgeführt. Die Gesamte Ansatzgröße wurde hälftig auf die beiden Reaktoren verteilt und die Reaktion wurde in den beiden Reaktoren mit einem zeitlichen Versatz von 3 h durchgeführt. Das Prozessabgas wurde in einer Sammelleitung vereint und der Absorptionsvorrichtung zugeführt, die ansonsten mit einer Regelung wie in Beispiel 1 betrieben wurde. Da hierbei die maximalen Massenströme an Prozessabgas aus den beiden Reaktoren nicht zeitgleich anfielen, fiel auch die maximale Gasbelastung der Absorptionskolonne geringer aus. Der beobachtete Phosgengehalt im Abgas schwankte zwischen 0,04 Vol.-% und 0,07 Vol.-%.

## Patentansprüche

1. Verfahren zur Herstellung eines Isocyanats umfassend die Schritte:
A) Umsetzung von mindestens einem Amin oder dessen Salz mit Phosgen, wobei ein flüssiges Reaktionsprodukt enthaltend das Isocyanat sowie ein erster gasförmiger Prozessabgasstrom enthaltend Chlorwasserstoff und Phosgen erhalten werden,
B) Auftrennung des gasförmigen ersten Prozessabgasstroms enthaltend Chlorwasserstoff, Phosgen, gegebenenfalls Lösungsmittel, gegebenenfalls leichtsiedende Nebenkomponenten und gegebenenfalls Inerte in mindestens einer Absorptionsvorrichtung, in der der erste Prozessabgasstrom mit einem Absorptionsmittel kontaktiert wird, wobei eine erste Phosgenlösung mit einem ersten Phosgengehalt und ein an Phosgen abgereicherter zweiter Abgasstrom mit einem zweiten Phosgengehalt erhalten werden,
C) gegebenenfalls Vermischen der in Schritt B) erhaltenen ersten Phosgenlösung mit zusätzlichem Phosgen und/oder Lösungsmittel unter Erhalt einer konditionierten zweiten Phosgenlösung,
D) gegebenenfalls Einsatz der ersten Phosgenlösung aus Schritt B) oder der konditionierten zweiten Phosgenlösung aus Schritt C) zur Phosgenierung eines Amins,
E) gegebenenfalls Isolierung des Isocyanats durch destillative Auftrennung des in Schritt A) erhaltenen flüssigen Reaktionsprodukts,
**dadurch gekennzeichnet, dass**
• der erste Phosgengehalt des in Schritt B) erhaltenen, an Phosgen abgereicherten zweiten Abgasstroms mit einem ersten Sollwert für diesen ersten Phosgengehalt verglichen wird und/oder der zweite Phosgengehalt der in Schritt B) erhaltenen Phosgenlösung ermittelt und mit einem zuvor festgelegten zweiten Sollwert für diesen zweiten Phosgengehalt verglichen wird und
• das Absorptionsmittel mit einem Massenstrom M1 in die Absorptionsvorrichtung eingebracht wird und dieser Massenstrom M1 einer Regelung unterliegt, wobei die Führungsgröße für den Massenstrom M1 vorzugsweise automatisch geändert wird, wenn der erste Phosgengehalt und/oder der zweite Phosgengehalt von ihrem jeweiligen Sollwert abweichen.

2. Verfahren gemäß Anspruch 1, **dadurch gekennzeichnet, dass** der Sollwert für den ersten Phosgengehalt im Bereich von 0,00 Vol.-% bis 2,00 Vol.-%, bevorzugt im Bereich von 0,01 Vol.-% bis 1,00 Vol.-% und besonders bevorzugt im Bereich von 0,02 Vol.-% bis 0,50 Vol.- % und ganz besonders bevorzugt im Bereich von 0,02 Vol.-% bis 0,2 Vol.-% liegt.

3. Verfahren gemäß Anspruch 1 oder 2, **dadurch gekennzeichnet, dass** der Sollwert für den zweiten Phosgengehalt im Bereich von 20 Gew.-% bis 70 Gew.- %, bevorzugt im Bereich von 30 Gew.-% bis 68 Gew.-% und besonders bevorzugt im Bereich von 42 Gew.-% bis 66 Gew.-% liegt.

4. Verfahren gemäß einem der Ansprüche 1 bis 3, **dadurch gekennzeichnet, dass** im Normalbetrieb der zweite Phosgengehalt durch Anpassung des Massenstroms M1 auf den zuvor festgelegten Sollwert geregelt wird und dass bei Überschreitung eines Schwellenwerts für den ersten Phosgengehalt, also einer Abweichung vom Normalbetrieb, der Massenstrom M1 erhöht wird.

5. Verfahren gemäß einem der Ansprüche 1 bis 4, **dadurch gekennzeichnet, dass** es sich bei dem mindestens einen Amin um ein Diamin, bevorzugt um 1,5-Diaminonaphthalin (NDA), 1,4-Diaminobenzol (pPDA), 1,5-Diaminopentan (PDA), Bis(p-aminocyclohexyl)methan (PACM), Hexahydrotoluylendiamin (H6TDA), inbesondere 2,4-Hexahydrotoluylendiamin und 2,6-Hexahydrotoluylendiamin, 1,3-Bis(aminomethyl)benzol (m-XDA) sowie Bis(aminomethyl)norbornan (NBDA) und Mischungen davon handelt.

6. Verfahren gemäß einem der Ansprüche 1 bis 5, **dadurch gekennzeichnet, dass** die Umsetzung des mindestens einen Amins oder dessen Salzes mit Phosgen in Gegenwart eines Lösungsmittels erfolgt.

7. Verfahren gemäß einem der Ansprüche 1 bis 6, **dadurch gekennzeichnet, dass** die Umsetzung des mindestens einen Amins oder dessen Salzes mit Phosgen in kondensierter Phase erfolgt.

8. Verfahren gemäß einem der Ansprüche 1 bis 7, **dadurch gekennzeichnet, dass** die Umsetzung des mindestens einen Amins oder dessen Salzes mit Phosgen chargenweise erfolgt.

9. Verfahren gemäß einem der Ansprüche 1 bis 8, **dadurch gekennzeichnet, dass** die Umsetzung in Schritt A) eine Basenphosgenierung von mindestens einem Amin mit Phosgen ist.

10. Verfahren gemäß einem der Ansprüche 8 bis 9, **dadurch gekennzeichnet, dass** die Umsetzung in Schritt A) in mindestens 2 parallel angeordneten Reaktoren erfolgt, wobei wenigstens ein Reaktor asynchron zu wenigstens einem der anderen Reaktoren betrieben wird.

11. Verfahren gemäß einem der Ansprüche 1 bis 10, **dadurch gekennzeichnet, dass** in dem mindestens einen Absorptionsschritt Chlorbenzol, o-Dichlorbenzol oder Mischungen dieser beiden Lösemittel, bevorzugt Chlorbenzol, als Absorbens eingesetzt wird.

12. Verfahren gemäß einem der Ansprüche 6 bis 11, **dadurch gekennzeichnet, dass** in dem mindestens einen Absorptionsschritt ein Lösemittel als Absorptionsmittel eingesetzt wird, das bevorzugt das Lösemittel ist, welches auch in der Umsetzung des mindestens einen Amins oder dessen Salzes zugegen ist.

13. Verfahren gemäß einem der Ansprüche 1 bis 12, **dadurch gekennzeichnet, dass** die Absorptionsvorrichtung einen Gaswäscher ausgewählt aus der Gruppe bestehend aus Füllkörperkolonnen, Packungskolonnen und Bodenkolonnen, bevorzugt bestehend aus Füllkörperkolonnen und Packungskolonnen, umfasst.

14. Vorrichtung zur Auftrennung von Abgasströmen aus einer Phosgenierungsreaktion umfassend mindestens eine Absorptionsvorrichtung zur Kontaktierung eines ersten Prozessabgasstroms enthaltend Chlorwasserstoff, Phosgen mit einem Absorptionsmittel sowie umfassend einen Computer zur Regelung der Auftrennung, **dadurch gekennzeichnet, dass** der Computer zur Kontrolle des Verfahrens nach einem der Ansprüche 1 bis 13 eingerichtet ist.
